# EUROPÄISCHE PATENTSCHRIFT

(11) **EP 0 773 211 B1**
(45) Veröffentlichungstag und Bekanntmachung des Hinweises auf die Patenterteilung: **12.01.2000**
(21) Anmeldenummer: 96117456.2
(22) Anmeldetag: 31.10.1996
(51) Int. Cl.: C07C 209/60, C07C 211/21

(54) **Verfahren zur Herstellung von primären Octadienylaminen**
Process for the preparation of primary octadienylamines
Procédé pour la préparation d'octadiénylamines primaires

(30) Priorität: 13.11.1995 DE 19542188; 28.05.1996 DE 19621303
(43) Veröffentlichungstag der Anmeldung: 14.05.1997
(73) Patentinhaber: BAYER AG, 51368 Leverkusen (DE)
(72) Erfinder: Driessen-Hölscher, Birgit, Dr., 52074 Aachen (DE); Keim, Wilhelm, Prof. Dr., 52074 Aachen (DE); Prinz, Thomas, 52070 Aachen (DE); Traenckner, Hans-Joachim, Dr.,, 51467 Bergisch Gladbach (DE); Jentsch, Jörg-Dietrich, Dr., 45468 Mühlheim a.d. Ruhr (DE)

(56) Entgegenhaltungen:
- DD-A- 129 779
- US-A- 4 104 471
- US-A- 4 130 590
- US-A- 4 186 148
- JOURNAL OF MOLECULAR CATALYSIS, Bd. 10, 1981, Seiten 107-114, XP000196826 JIRO TSUJI ET AL.: "Palladium-catalyzed telomerization of butadiene with ammonia"
- DATABASE WPI Section Ch, Week 9331 Derwent Publications Ltd., London, GB; Class A60, AN 93-249126 XP002025480 & SU 1 754 705 A (UNIV IRKUT ZHDANOV) , 15.August 1992
- CHEMICAL ABSTRACTS, vol. 74, no. 25, 21.Juni 1971 Columbus, Ohio, US; abstract no. 140785n, TSUJI, JIRO ET AL.: "Syntheses of long-chain amines by palladium-catalyzed telomerization of butadiene" Seite 513; Spalte 1; XP002025479 & J. CHEM. SOC. D., Bd. 7, 1971, Seite 345

## Beschreibung

Die vorliegende Erfindung betrifft ein Verfahren zur Herstellung primärer Octadienylamine aus Ammoniak und 1,3-Butadien.

Octadienylamine sind z.B. als Zwischenprodukte zur Herstellung von Octylaminen verwendbar, die ihrerseits z.B. zur Herstellung von Weichspülern, Korrosionsinhibitoren, Flotationshilfsmittel und Emulgatoren benötigt werden. Für diesen Zweck wird Octyl-1-amin bevorzugt. Deshalb haben solche Octadienylamine die größte Bedeutung, die sich in Octyl-1-amin überführen lassen. Das sind insbesondere Octa-2,7-dienyl-1-amin und Octa-1,7-dienyl-3-amin.

Es ist bekannt, daß durch Telomerisation von Ammoniak mit 1,3-Butadien ein Gemisch von Octadienylaminen hergestellt werden kann. Bei dieser Telomerisation werden bisher jedoch überwiegend stets bedeutende Anteile an tertiären Aminen wie Tri-(octa-2,7-dienyl)-amin und Di-(octa-1,7-dienyl-3)-(octa-2,7-dienyl-1)-amin erhalten, weil mit zunehmendem Alkylierungsgrad die Basizität des Stickstoffs steigt und deshalb im vorliegenden Fall sekundäre Amine reaktionsfähiger als primäre Amine und primäre Amine reaktionsfähiger als Ammoniak sind.

Die bisher genannten Octadienylamine entsprechen den folgenden Formeln (I) bis (IV) und werden im folgenden auch mit den hier angegebenen römischen Zahlen bezeichnet.

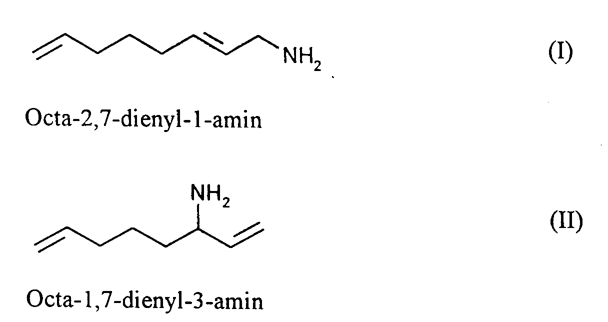

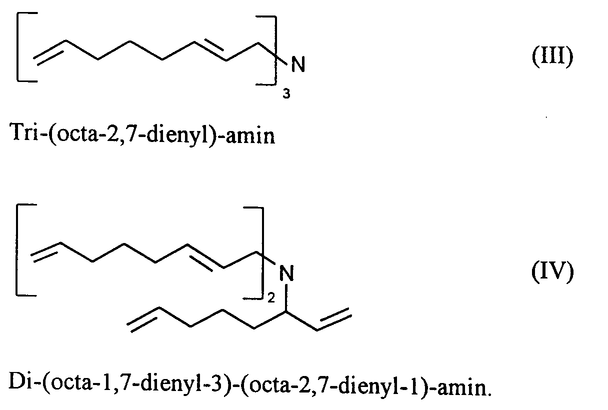

Es ist bekannt, daß die Telomerisation von Butadien mit Ammoniak nur in Anwesenheit von Wasser zu zufriedenstellenden Ausbeuten führt (J. Chem. Soc. Chem. Comm. 1971, 345 und J. Mol. Catal. 1981, 10, 107). Ammoniak wird deshalb in Form einer wäßrigen Lösung in die Reaktion eingesetzt. Mit einem Katalysatorsystem, das Palladiumacetat und Triphenylphosphan im molaren Verhältnis 1:3,5 enthält, werden die Amine (III) und (IV) in insgesamt 100 %iger Ausbeute, bezogen auf Ammoniak, erhalten.

Desweiteren ist die Telomerisation von 1,3-Butadien mit verschiedenen Ammoniumsalzen beschrieben (Neftekhimiya, 1979, 19, 468). Als Katalysator dient ein in situ-System aus Palladiumacetylacetonat, Triphenylphosphan und Triethylaluminium im Verhältnis 1:2:3. Beim Einsatz von Ammoniumhydrogencarbonat als Ammoniumsalz erhält man ein Produktgemisch, das mindestens 5 Komponenten, darunter das Amin (II), enthält. Das unerwünschte tertiäre Amin (III) und ein stickstofffreies Dimeres des Butadiens entstehen dabei zu jeweils 25 %. Bei Zusatz von Trifluoressigsäure führt diese Telomerisation mit einer Ausbeute von 25 % und einer Selektivität von 100 % zum unerwünschten tertiären Amin (III). Beim Einsatz von Ammoniumtartrat anstelle von Ammoniumhydrogencarbonat enthält das Produktgemisch nur noch 3 Komponenten, wovon aber 59 Gew.-% stickstofffreie Butadienoligomere sind. Bei keinem dieser Verfahren kann das lineare primäre Octadienylamin (I) aus dem Reaktionsgemisch isoliert werden.

Aus den US-Patenten 4 100 194 und 4 130 590 ist bekannt, daß sekundäre und tertiäre Octadienylamine unter Verwendung des Katalysatorsystems Palladiumacetat/Diisopropyl(phenyl)phosphonit aus Butadien und Ammoniak hergestellt werden können. Bei einer Temperatur von 70°C und einer Reaktionszeit von einer Stunde wird ein Umsatz von 98 % beobachtet. Bei Zusatz von Trifluoressigsäure wird auch hier die ausschließliche Bildung des unerwünschten tertiären Amins (III) beobachtet.

Die Durchführung von homogenen, durch Übergangsmetall-Verbindungen katalysierten Reaktionen in zwei nicht mischbaren Phasen erlaubt eine einfache Abtrennung des Katalysators von den Reaktionsprodukten (Angew. Chem. 1993, 105, 1588 und Adv. Organomet. Chem. 1992, 34, 219). Auch Telomerisationsreaktionen sind schon in Zweiphasensystemen durchgeführt worden, z.B. die Telomerisation von Phthalsäure mit 1,3-Butadien, wobei die eine Phase aus Dimethylsulfoxid und die andere aus Isooctan gebildet war (Erdöl und Kohle 1976, 29, 31).

Aus der DE-OS 2 733 516 ist die Telomerisation von Dienen in einem Zweiphasensystem bekannt. Dabei wurde erstmals Wasser als Lösungsmittel für den Katalysator benutzt und die Verwendung sulfonierter Phosphane beschrieben, die nach Komplexbildung mit dem Übergangsmetall dessen Wasserlöslichkeit bewirken. Als Nukleophile wurden Wasser, Alkohole, Phenole, Säuren, Amine, CH-aktive Substanzen und Silanole eingesetzt. Bei der Umsetzung von Butadien mit Aminen ist nur die Umsetzung mit Diethylamin exemplifiziert, wobei 1-Diethylaminobuten-2 und 1-N-Diethylaminooctadien-2,7 als Produkte entstehen (siehe Beispiele 25 und 26). Es werden also aus einem sekundären Amin tertiäre Amine erhalten. Da, wie oben aufgeführt, die Reaktivität von sekundären Aminen gegenüber Butadien wesentlich höher ist als diejenige von Ammoniak kann diese DE-OS das erfindungsgemäße Verfahren nicht nahelegen.

Aus mehreren Patenten ist die Telomerisation von Butadien und Wasser zu Octadienolen in einem Zweiphasensystem bekannt (siehe US-Patente 4 356 333 und 4 417 079 sowie EP-OS 436 226). Die Reaktion wird dabei in einer wäßrigen Sulfolan-Lösung durchgeführt, aus der sich die gebildeten Octadienole als zweite Phase abscheiden. Der Palladiumkatalysator wird durch monosulfoniertes Triphenylphosphan (TPPMS) in der Sulfolan-Phase zurückgehalten.

Wasserlösliche quartäre Ammoniumphosphine als Liganden für Übergangsmetalle sind bei der Telomerisation von Dienen mit Methanol unter Zweiphasenbedingungen beschrieben (J. Mol. Catal., 1990, 59, 1).

Das FR-Patent 2 693 188 beschreibt die Telomerisation von Saccharose mit Butadien in wäßriger Lösung unter Verwendung eines Katalysatorsystems aus Palladiumacetat und trisulfoniertem Triphenylphosphan (TPPTS). Der Umsatz bezüglich Saccharose beträgt 96 %. Es entstehen aber verschiedene Octadienylether unterschiedlichen Alkylierungsgrades, wobei die Diether, Triether und Tetraether überwiegen.

Aus einer kürzlich erschienenen Publikation ist die Telomerisation von Butadien und Wasser im Zweiphasensystem mit einem Trialkylamin als Additiv bekannt (J. Mol. Catal. A: Chemical, 1995, 97, 29). Das Katalysatorsystem besteht aus einem Palladiumsalz und TPPMS oder TPPTS. Das Reaktionsgemisch enthält bis zu 5 Telomerisationsprodukte (Alkohole, Olefine und Ether). Die Reaktion ist also unselektiv.

Zusammenfassend ist festzustellen, daß Zweiphasentelomerisationen von Butadien und Ammoniak noch nicht beschrieben wurden und die literaturbekannten Verfahren für die Zweiphasentelomerisationen mit Butadien unselektiv verlaufen.

Der vorliegenden Erfindung liegt die Aufgabe zugrunde, die Telomerisation von Butadien und Ammoniak so durchzuführen, daß möglichst selektiv die primären Octadienylamine (I) und (II) entstehen und die Bildung unerwünschter sekundärer und tertiärer Octadienylamine möglichst weitgehend verhindert wird. Gemäß den bisher bekannten Telomerisierungsverfahren erhält man das Amin (I) praktisch nicht und das Amin (II) nur mit größeren Anteilen unerwünschter Produkte.

Es wurde nun ein Verfahren zur selektiven Herstellung von Octa-2,7-dienyl-1-amin und Octa-1,7-dienyl-3-amin gefunden, das dadurch gekennzeichnet ist, daß man Butadien und Ammoniak in einem Zweiphasensystem telomerisiert, wobei man den Katalysator in wäßriger Phase anwendet und als zweite Phase ein organisches Medium einsetzt, von dem sich bei 20°C in 100 g Wasser weniger als 5 g lösen.

Als Katalysatoren kommen z.B. Gemische von Palladiumverbindungen und diese besser wasserlöslich machenden Phosphorverbindungen in Frage. In den Palladiumverbindungen kann das Palladium in den Oxidationsstufen Null, +1 und/oder +2 vorliegen. Besonders geeignet ist Palladium(II)-acetat. Als Phosphorverbindungen kommen z.B. sulfonierte Triphenylphosphane in der Säure- und/oder Salzform in Frage. Geeignet sind beispielsweise mono-, di- und/oder trisulfonierte Triphenylphosphane in der Säure- und/oder Salzform. Besonders bevorzugt ist trisulfoniertes Triphenylphosphan in Form des Trinatriumsalzes. Auch weitere wasserlöslich machende Phosphorverbindungen kommen in Betracht, z.B. Phosphane, die Carboxyl- oder Hydroxylgruppen enthalten.

Bezogen auf 1 g Wasser kann man den Palladium-Katalysator z.B. in Mengen von 7,5·10⁻⁵ bis 0,75 mmol Pd und bezogen auf 1 mol Palladiumkatalysator die besser wasserlöslich machende Phosphorverbindung z.B. in Mengen von 1 bis 10 mol einsetzen.

Als organisches Medium kommt insbesondere eines in Frage, von dem sich bei 20°C in 100 g Wasser weniger als 3 g lösen. Geeignet sind beispielsweise aliphatische und aromatische Kohlenwasserstoffe und chlorierte aliphatische und aromatische Kohlenwasserstoffe wie C₃-C₃₀-Alkane, Benzol, mono-, di- und/oder tri-C₁-C₄-Alkylbenzole, mono- und polychlorierte C₁-C₁₂-Alkane, mono-, di- und trichlorierte Benzole und mono-, di- und trichlorierte C₁-C₄-Alkylbenzole. Man kann als organisches Medium auch Butadien verwenden. In diesem Falle sind mindestens 0,5 Mol Butadien pro Mol Ammoniak einzusetzen. Bevorzugt sind hierbei 0,7 bis 100 Mol Butadien pro Mol Ammoniak. Bevorzugte organische Medien sind Dichlormethan, Dichlorethan, Toluol, Pentan und Butadien. Man kann auch Gemische verschiedener organischer Medien einsetzen.

Ammoniak kann man in beliebiger Form einsetzen. Vorzugsweise verwendet man wäßrige Lösungen von Ammoniak, beispielsweise 5 bis 35 gew.-%ige wäßrige Ammoniaklösungen oder reinen Ammoniak (Handelsprodukt).

Bezogen auf 100 ml organisches Medium kann man z.B. 0,1 bis 10 000 ml Wasser einsetzen, wobei das Wasser z.B. als solches und/oder in Form einer wäßrigen Ammoniaklösung eingebracht werden kann. Wenn man ein von Butadien verschiedenes organisches Medium einsetzt, kann man es in beliebigen Mengen einsetzen, wobei bezogen auf 1 mol Butadien 80 bis 500 ml eines solchen Lösungsmittels bevorzugt sind.

Das erfindungsgemäße Verfahren kann z.B. bei Temperaturen von 30 bis 150°C und Drucken im Bereich 1 bis 150 bar durchgeführt werden. Vorzugsweise arbeitet man im geschlossenen Gefäß bei dem sich bei der jeweiligen Reaktionstemperatur von selbst einstellenden Druck.

Die Reaktionszeit beträgt im allgemeinen 15 Minuten bis 4 Stunden. Die für den einzelnen Fall günstigste Reaktionszeit kann man gegebenenfalls durch routinemäßige Reihenversuche ermitteln, wobei zu beachten ist, daß mit längerer Reaktionszeit i.a. der Umsatz des Butadiens zunimmt, aber auch die Bildung unerwünschter sekundärer und tertiärer Amine. Das ist besonders ausgeprägt, wenn man mit Butadien und ohne andere organische Medien arbeitet. Es ist vorteilhaft, während der Umsetzung zu rühren oder das Reaktionsgefäß zu schütteln.

Das nach der Umsetzung vorliegende Reaktionsgemisch kann man auf einfache Weise aufarbeiten. Beispielsweise kann man so vorgehen, daß man die wäßrige Phase von der organischen Phase trennt, die wäßrige Phase mit einem mit Wasser nicht oder nur wenig mischbaren organischen Lösungsmittel wäscht, vorzugsweise mit dem in der Reaktion eingesetzten organischen Lösungsmittel, die Waschflüssigkeit mit der abgetrennten organischen Phase vereinigt und daraus die erhaltenen Telomerisationsprodukte abtrennt und aufrennt, z.B. durch Destillation.

Wenn man die Umsetzung mit Butadien und ohne zusätzliches Lösungsmittel durchgeführt hat, ist es i.a. ausreichend, zur Aufarbeitung zunächst nicht umgesetzes Butadien oder nicht umgesetzten Ammoniak zu entfernen, z.B. durch Druckentspannung und dann die organische Phase abzutrennen. Durch Destillation der organischen Phase kann man dann die erhaltenen Telomerisationsprodukte auftrennen.

Bei einer bevorzugten Ausführungsform des erfindungsgemäßen Verfahrens legt man den Katalysator und Wasser bei Raumtemperatur in einem Autoklaven vor, fügt wäßrige Ammoniaklösung und organisches Lösungsmittel zu, kondensiert dann eine abgemessene Menge Butadien und gegebenenfalls Ammoniak bei tiefer Temperatur in den Autoklaven hinein und erhitzt ihn nach dem Verschließen auf die Reaktionstemperatur. Nach Beendigung der Reaktion und gegebenenfalls eines Nachrührens bringt man abschließend den Autoklaven auf Raumtemperatur, entspannt ihn und arbeitet das vorliegende Reaktionsgemisch wie oben beschrieben auf.

Das erfindungsgemäße Verfahren kann man diskontinuierlich oder kontinuierlich durchführen.

Das erfindungsgemäße Verfahren hat eine Reihe von Vorteilen. So enthält das Telomerisationsprodukt wesentlich höhere Anteile an den Octadienylaminen (I) und (II) als bei anderen Verfahren. Die Selektivität der Bildung von (I) und (II) liegt beim erfindungsgemäßen Verfahren im allgemeinen zwischen 70 und nahezu 100 %. Die Ausbeuten von (I) bezogen auf eingesetztes Butadien sind beim erfindungsgemäßen Verfahren vergleichbar bis besser als bei anderen Verfahren. Bei zu geringen Butadienmengen steigt der Anteil unerwünschter sekundärer Amine im Telomerisationsprodukt stark an (siehe Vergleichsbeispiel 1). Bei Verwendung von ungeeigneten organischen Lösungsmitteln geht die Selektivität der Bildung von (I) und (II) auf unter 35 % zurück und es finden nur ganz geringe Umsätze statt (siehe Vergleichsbeispiel 2).

Insbesondere sind beim erfindungsgemäßen Verfahren die Selektivitäten und Ausbeuten an (I) und (II) wesentlich höher als bei bekannten Verfahren zur Herstellung von Octadienylaminen aus Ammoniak oder Ammoniumverbindungen und Butadien.

Beim erfindungsgemäßen Verfahren befindet sich nach der Reaktion der Katalysator praktisch ausschließlich in der wäßrigen Phase. Er ist deshalb leicht von den in der organischen Phase befindlichen Reaktionsprodukten abtrennbar und kann auf einfache Weise recyclisiert werden.

Diese Vorteile sind ausgesprochen überraschend, da es bisher nicht gelungen ist, die Octadienylamine (I) und (II) selektiv aus Butadien und Ammoniak oder Ammoniumverbindungen herzustellen. Bekannte 2-Phasen-Telomerisationsreaktionen von Butadien mit anderen Nukleophilen, z.B. mit Saccharose oder Wasser, sind im Gegensatz zum erfindungsgemäßen Verfahren unselektiv.

### Beispiele

### Beispiel 1

Eine Katalysatorlösung bestehend aus 0,15 mmol Palladium(II)-acetat und 0,6 mmol TPPTS in 5 ml Wasser wurde in einem mit Inertgas gespülten Autoklaven vorgelegt und 20 ml einer 27,13 molaren wäßrigen Ammoniaklösung und 12,5 ml Dichlormethan zugegeben. Der Autoklav wurde gewogen und dann in einem Ethanol/Trockeneis-Bad gekühlt. Nun wurden 5,8 ml flüssiges Butadien zugesetzt und der Autoklav im Verlauf einer Stunde wieder auf Raumtemperatur gebracht. Er wurde nochmals zur genauen Bestimmung der Butadieneinwaage gewogen und dann in ein auf 100°C vorgeheiztes Ölbad gehängt. Nach Ablauf einer Reaktionszeit von 1,5 Stunden wurde der Autoklav mit Stickstoff auf Raumtemperatur gekühlt, dann entspannt und geöffnet. Der Inhalt wurde in einen Scheidetrichter überführt und die Phasen getrennt. Die wäßrige Phase wurde mit 10 ml Dichlormethan extrahiert. Durch die vereinigten organischen Phasen wurde zur Entfernung restlichen Butadiens Argon geblasen. Abschließend wurde den vereinigten organischen Phasen eine Probe entnommen, über einem Molekularsieb getrocknet und gaschromatographisch untersucht.

Die Ergebnisse sind aus Tabelle 1 ersichtlich.

### Beispiele 2 bis 5

Es wurde verfahren wie bei Beispiel 1, jedoch wurden die Reaktionstemperaturen und -zeiten variiert. Die Ergebnisse sind aus Tabelle 1 ersichtlich.

**Tabelle 1**

| Bsp. Nr. | Reaktionstemperatur (°C) | Reaktions zeit (Stunden) | Selektivität der Bildung von | | | Ausbeute an (I) bezogen auf Butadien |
|---|---|---|---|---|---|---|
| | | | (I) | (II) | (I)+(II) | |
| 1 | 100 | 1,5 | 42 % | 28 % | 70 % | 28 % |
| 2 | 80 | 1 | 54 % | 39 % | 93 % | 17 % |
| 3 | 80 | 1,5 | 51 % | 40 % | 91 % | 16 % |
| 4 | 60 | 1 | 56 % | 40 % | 96 % | 13 % |
| 5 | 60 | 2 | 53 % | 41 % | 94 % | 13 % |

Die Selektivität der Bildung von sekundären Aminen lag bei 1 bis 7 %.

### Beispiele 6 bis 8 und Vergleichsbeispiele 1 bis 2

Es wurde bei 80°C und entsprechend Beispiel 1, jedoch mit verschiedenen organischen Lösungsmitteln oder in Abwesenheit eines organischen Lösungsmittels gearbeitet. Die Ergebnisse sind aus Tabelle 2 ersichtlich.

**Tabelle 2**

| Bsp. Nr. | Reaktionszeit Stunden | Lösungsmittel | Selektivität der Bildung von | | | | Ausbeute an (I) bezogen auf Butadien |
|---|---|---|---|---|---|---|---|
| | | | (I) | (II) | (I)+(II) | sek.Amin | |
| 6 | 1,5 | CH₂Cl₂ | 51 % | 40 % | 91 % | 2 % | 16 % |
| 7 | 1,5 | C₂H₄Cl₂ | 45 % | 34 % | 79 % | 4 % | 19 % |
| 8 | 1,5 | Toluol | 50 % | 38 % | 88 % | 6 % | 30 % |
| 9 | 1,5 | Pentan | 46 % | 33 % | 79 % | 16 % | 29 % |
| V1 | 1 | - | 34 % | 26 % | 60 % | 30 % | 15 % |
| V2 | 1,5 | Essigester | 15 % | 19 % | 34 % | n.b. | <1 % |
| n.b. = nicht bestimmt; V1 und V2 sind Vergleichsversuche. | | | | | | | |

V1 zeigt, daß bei Abwesenheit eines organischen Lösungsmittels die Selektivität der Bildung von (I)+(II) deutlich abnimmt und die Selektivität der Bildung von sekundären Alpinen stark zunimmt. V2 zeigt, daß beim Einsatz eines organischen Lösungsmittels, das nicht ausreichend wenig mit Wasser mischbar ist die Selektivität der Bildung von (I)+(II) und die Ausbeute an (I) sehr stark abnehmen.

### Beispiele 10 bis 12

Es wurde bei 80°C entsprechend Beispiel 1, jedoch mit unterschiedlichen Katalysatorkonzentrationen und unterschiedlichen Verhältnissen von Palladiumkatalysator : Butadien gearbeitet. Die Ergebnisse sind aus Tabelle 3 ersichtlich.

**Tabelle 3**

| Bsp. Nr. | Konzentration des Katalysators (mol/l) | Molverhältnis Pd:Butadien | Selektivität der Bildung von | | | Ausbeute an (I) bezogen auf Butadien |
|---|---|---|---|---|---|---|
| | | | (I) | (II) | (I)+(II) | |
| 10 | 1,2x10⁻² | 1:260 | 45 % | 43 % | 88 % | 7 % |
| 11 | 6x10⁻³ | 1:520 | 49 % | 41 % | 90 % | 17 % |
| 12 | 3x10⁻³ | 1:947 | 51 % | 41 % | 92 % | 13 % |

### Beispiel 13

In einem Schlenkrohr wurden zunächst 1,5 mmol Palladium(II)-acetat vorgelegt und in 20 ml 25 gew.-%iger wäßriger Ammoniaklösung gelöst. Anschließend wurden 4,5 mmol TPPTS hinzugefügt.

In einem 900 ml-Rührkessel wurden 200 ml Wasser vorgelegt und die im Schlenkrohr vorbereitete Katalysatorlösung dazugegeben. Unter Rühren wurden 45 g Ammoniak und anschließend 120 g Butadien zudosiert. Man erwärmte den Autoklaven unter heftigem Rühren auf 60°C und nahm nach 45 und 75 Minuten je eine Probe. Die entnommenen Proben (ca. 3 ml) wurden jeweils mit 1 ml Toluol versetzt. Nach erfolgter Phasentrennung wurde die organische Phase über einem Molsieb getrocknet und der gaschromatographischen Analyse zugeführt.

Die Ergebnisse sind aus Tabelle 4 ersichtlich.

### Beispiel 14

Es wurde verfahren wie in Beispiel 13, jedoch wurde das Reaktionsgemisch auf 80°C erwärmt und eine Probe nach 30 Minuten entnommen und analysiert. Die Ergebnisse sind aus Tabelle 4 ersichtlich.

**Tabelle 4**

| Bsp.-Nr. | Reaktions | | Selektivität der Bildung von | | | Umsatz von Butadien |
|---|---|---|---|---|---|---|
| | temp. | zeit | (I) + (II) | sek.-Amin | tert.-Amin | |
| 13 | 60°C | 45 min | 94 % | 5 % | - | 6 % |
| 13 | 60°C | 75 min | 92 % | 7 % | - | 20,5 % |
| 14 | 80°C | 30 min | 88 % | 10 % | - | 39 % |

## Patentansprüche

1. Verfahren zur selektiven Herstellung von Octa-2,7-dienyl-1-amin und Octa-1,7-dienyl-3-amin, dadurch gekennzeichnet, daß man Butadien und Ammoniak in einem Zweiphasensystem telomerisiert, wobei man den Katalysator in wäßriger Phase anwendet und als zweite Phase ein organisches Medium einsetzt, von dem sich bei 20°C in 100 g Wasser weniger als 5 g lösen.

2. Verfahren nach Anspruch 1, dadurch gekennzeichnet, daß als Katalysator Gemische von Palladiumverbindungen und diese besser wasserlöslich machenden Phosphorverbindungen eingesetzt werden.

3. Verfahren nach Anspruch 1 und 2, dadurch gekennzeichnet, daß als Katalysator 7,5·10⁻⁵ bis 0,75 mMol Palladium (bezogen auf 1 g Wasser) und 1 bis 10 mol einer besser wasserlöslich machenden Verbindung (bezogen auf 1 mol Palladium) eingesetzt werden.

4. Verfahren nach Anspruch 1 bis 3, dadurch gekennzeichnet, daß als organisches Medien ein solches eingesetzt wird, von dem sich bei 20°C in 100 g Wasser weniger als 3 g lösen.

5. Verfahren nach Anspruch 1 bis 4, dadurch gekennzeichnet, daß als organisches Medium C₃-C₃₀-Alkane, Benzol, mono-, di- und/oder tri-C₁-C₄-Alkylbenzole, mono- und/oder polychlorierte C₁-C₁₂-Alkane, mono-, di- und/oder trichlorierte Benzole und/oder mono-, di- und/oder trichlorierte C₁-C₄-Alkylbenzole oder Butadien oder Gemische verschiedener organischer Medien eingesetzt werden.

6. Verfahren nach Anspruch 1 bis 5, dadurch gekennzeichnet, daß Ammoniak als 5 bis 35 gew.-%ige wäßrige Lösung eingesetzt wird.

7. Verfahren nach Anspruch 1 bis 6, dadurch gekennzeichnet, daß auf 100 ml organisches Medium 0,1 bis 10 000 ml Wasser und auf 1 mol Butadien 80 bis 500 ml organisches Lösungsmittel eingesetzt werden.

8. Verfahren nach Anspruch 1 bis 5, dadurch gekennzeichnet, daß man mindestens 0,5 Mol Butadien pro Mol Ammoniak einsetzt.

9. Verfahren nach Anspruch 1 bis 8, dadurch gekennzeichnet, daß man es bei 30 bis 150°C und 1 bis 150 bar durchführt.

10. Verfahren nach Anspruch 1 bis 9, dadurch gekennzeichnet, daß man das Reaktionsgemisch aufarbeitet, indem man die wäßrige Phase von der organischen Phase trennt, die wäßrige Phase mit einem mit Wasser nicht oder nur wenig mischbaren organischen Lösungsmittel wäscht, die Waschflüssigkeit mit der abgetrennten organischen Phase vereinigt und daraus die erhaltenen Telomerisationsprodukte abtrennt und auftrennt.

## Claims

1. Process for the selective preparation of octa-2,7-dienyl-1-amine and octa-1,7-dienyl-3-amine, characterized in that butadiene and ammonia are telomerized in a two-phase system, the catalyst being used in the aqueous phase and the organic medium employed as the second phase being one of which less than 5g dissolve in 100g of water at 20°C.

2. Process according to Claim 1, characterized in that a mixture of a palladium compound and a phosphorus compound which renders this more water-soluble is employed as the catalyst.

3. Process according to Claims 1 and 2, characterized in that 7.5×10⁵ to 0.75 mmol of palladium (per g of water) and 1 to 10 mol of a compound which renders the palladium more water-soluble (per mol of palladium) are employed as the catalyst.

4. Process according to Claims 1 to 3, characterized in that the organic medium employed is one of which less than 3 g dissolve in 100 g of water at 20°C.

5. Process according to Claims 1 to 4, characterized in that the C₃-C₃₀-alkanes, benzene, mono-, di- and/or tri-C₁-C₄-alkylbenzenes, mono- and/or polychlorinated C₁-C₁₂-alkanes, mono-, di- and/or trichlorinated benzenes and/or mono-, di- and/or trichlorinated C₁-C₄-alkylbenzenes or butadiene or mixtures of different organic media are employed as the organic medium.

6. Process according to Claims 1 to 5, characterized in that ammonia is employed as a 5 to 35% strength by weight aqueous solution.

7. Process according to Claims 1 to 6, characterized in that 0.1 to 10,000 ml of water per 100 ml of organic medium and 80 to 500 ml of organic solvent per mole of butadiene are employed.

8. Process according to Claims 1 to 5, characterized in that at least 0.5 mol of butadiene is employed per mole of ammonia.

9. Process according to Claims 1 to 8, which is carried out at 30 to 150°C under 1 to 150 bar.

10. Process according to Claims 1 to 9, characterized in that the reaction mixture is worked up by separating the aqueous phase from the organic phase, washing the aqueous phase with an organic solvent which is immiscible or only slightly miscible with water, combining the wash liquid with the organic phase which has been separated off and separating off the resulting telomerization products therefrom and separating them.

## Revendications

1. Procédé de préparation sélective d'octa-2,7-diényl-1-amine et d'octa-1,7-diényl-3-amine, caractérisé en ce que l'on télomérise le butadiène et l'ammoniaque dans un système biphasique, où l'on emploie le catalyseur en phase aqueuse et comme deuxième phase, on met en oeuvre un milieu organique, dont, à 20°C et dans 100 g d'eau, moins de 5 g sont solubles.

2. Procédé suivant la revendication 1, caractérisé en ce que l'on met en oeuvre comme catalyseur, des mélanges de composés du palladium et des composés du phosphore améliorant leur solubilité dans l'eau.

3. Procédé suivant les revendications 1 et 2, caractérisé en ce que l'on met en oeuvre comme catalyseur, 7,5.10⁻⁵ à 0,75 mmole de palladium (sur base de 1 g d'eau) et 1 à 10 moles d'un composé améliorant sa solubilité dans l'eau (sur base de 1 mole de palladium).

4. Procédé suivant les revendications 1 à 3, caractérisé en ce que l'on met en oeuvre comme milieu organique, celui dont, à 20°C et dans 100 g d'eau, moins de 3 g sont solubles.

5. Procédé suivant les revendications 1 à 4, caractérisé en ce que l'on met en oeuvre comme milieu organique, des alcanes en C₃-C₃₀, le benzène, des mono-, di- et/ou tri(alcoyl en C₁-C₄)benzènes, des alcanes en C₁-C₁₂ mono- et/ou polychlorés, des benzènes mono-, di- et trichlorés et/ou des (alcoyl en C₁-C₄)benzènes mono-, di- et trichlorés ou du butadiène ou des mélanges de différents milieux organiques.

6. Procédé suivant les revendications 1 à 5, caractérisé en ce que l'ammoniaque est mis en oeuvre sous forme d'une solution aqueuse allant de 5 à 35% en poids.

7. Procédé suivant les revendications 1 à 6, caractérisé en ce que l'on met en oeuvre, pour 100 ml de milieu organique, de 0,1 à 10 000 ml d'eau et pour 1 mole de butadiène, de 80 à 500 ml de solvant organique.

8. Procédé suivant les revendications 1 à 5, caractérisé en ce que l'on met en oeuvre, au moins 0,5 mole de butadiène par mole d'ammoniac.

9. Procédé suivant les revendications 1 à 8, caractérisé en ce qu'on le réalise entre 30 et 150°C et entre 1 et 150 bar.

10. Procédé suivant les revendications 1 à 9, caractérisé en ce que l'on traite le mélange réactionnel en séparant la phase aqueuse de la phase organique, en lavant la phase aqueuse avec un solvant organique non ou seulement peu miscible dans l'eau, en combinant le liquide de lavage avec la phase organique séparée et en en séparant et isolant les produits obtenus de télomérisation.
